# EUROPEAN PATENT APPLICATION

(11) **EP 4 768 010 A1**
(43) Date of publication of application: **01.07.2026**
(21) Application number: 25217245.7
(22) Date of filing: 20.11.2025
(51) Int. Cl.: A61K 8/02, A61K 8/27, A61K 8/37, A61K 8/85, A61Q 1/00, A61Q 1/12, A61Q 9/02, A61Q 15/00, A61Q 17/04, A61Q 19/10

(54) **PARTICLES, COSMETICS, AND METHOD FOR PRODUCING PARTICLES**

(30) Priority: 26.12.2024 JP 2024229822; 20.08.2025 JP 2025136938
(71) Applicant: ETRIA Co., Ltd., Yokohama, Kanagawa 220-0011 (JP)
(72) Inventor: SATO, Yuichi, Yokohama, 220-0011 (JP); INOUE, Ryota, Yokohama, 220-0011 (JP)
(74) Representative: Marks & Clerk LLP

(57) **Abstract**

Particles including a biodegradable resin and a metal oxide, in which a ratio of an absorption amount of oleic acid to an absorption amount of triglyceride in the particles is 1.5 or more.

## Description

### BACKGROUND

### Technical Field

The present disclosure relates to particles, cosmetics, and a method for producing particles.

### Related Art

It is known that free fatty acids, which are components derived from sebum secreted by a human body, may cause body odor and unpleasant smells, and may also lead to temporal shine in makeup. From the viewpoint of improving the cosmetic retention of makeup cosmetics, materials and methods for removing free fatty acids from a skin surface have been investigated.

Heretofore, there are provided measures including a method including incorporating an oil-absorbing powder such as silica gel into cosmetics to adsorb sebum components, or a method including replacing powders used in makeup cosmetics with fluorine-treated powders having high oil repellency. Film-forming polymers such as acrylic-silicone based graft polymers are known for improving cosmetic retention.

Zinc oxide coated materials in which a base is coated with amorphous zinc oxide have been proposed, and powders with good spreadability not impairing fatty acid solidification ability, and skin external preparations using such powders, have been reported (for example, see Japanese Unexamined Patent Application Publication No. H9-227792). Interlayer metal inclusion compounds selectively adsorbing only free fatty acids, or compounds including one or more oxides and/or hydroxides thereof intercalated between the layers of clay minerals, have been reported (for example, see Japanese Unexamined Patent Application Publication No. H10-87420). Composite powders having a hydroxyapatite layer on the particle surface of a base and a zinc oxide layer on a surface of the resultant base have been proposed (for example, see Japanese Unexamined Patent Application Publication No. 2002-20218).

### SUMMARY

An object of the present disclosure is to provide particles that can selectively adsorb free fatty acids and are excellent in cosmetic retention.

Particles according to embodiments of the present disclosure as means for solving the aforementioned problems are particles including a biodegradable resin and a metal oxide, in which a ratio of an absorption amount of oleic acid to an absorption amount of triglyceride in the particles is 1.5 or more.

According to the present disclosure, particles that can selectively adsorb free fatty acids and are excellent in cosmetic retention are provided.

### DETAILED DESCRIPTION

In describing embodiments, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result.

Embodiments of the present disclosure are described below. As used herein, the singular forms "a," "an," and "the" are intended to include the plural forms as well, unless the context clearly indicates otherwise.

### (Particles)

Particles according to the present embodiment include a biodegradable resin and a metal oxide, and further optionally include other materials. A ratio of an absorption amount of oleic acid to an absorption amount of triglyceride in the particles is 1.5 or more. According to the particles of the present embodiment, free fatty acids can be selectively adsorbed, and the particles provide excellent cosmetic retention. Therefore, the particles can be suitably used for applications such as sebum control particles and cosmetics.

The particles according to the present embodiment are based on finding of problems in conventional technologies described below.

That is, when conventional oil-absorbing powders such as silica gel are used, oily components other than free fatty acids, such as triglycerides, which are important for an emollient aspect of the skin, are also adsorbed.

Therefore, blending a sufficient amount for completely adsorbing free fatty acids might lead to shortage of an emollient component, and thus, blending the sufficient amount is difficult. Another problem is that when such powders adsorb oily components, the powders exhibit a luster due to wetting by the oily components, resulting in a shiny makeup finish and a soiled makeup.

Conventional fluorine-treated powders have a problem that the powders resist wetting by sebum components, but the strong oil repellency causes the powders to slip over sebum leading to creasing. That is, there has been a demand for cosmetics material selectively adsorbing only free fatty acids.

Conventional acrylic-silicone based graft polymers can be produced by radical polymerization of a dimethylpolysiloxane compound having radical polymerizability at the terminal of molecular chain segments and a radical polymerizable monomer formed mainly of acrylate or methacrylate. The acrylic-silicone based graft polymers form a cosmetic film with excellent water and oil resistance and have been put to practical use in non-aqueous foundations. However, cosmetics using film-forming polymers such as acrylic-silicone based graft polymers still have challenges in terms of skin physiology, considering healthy biological reactions on the skin surface from the viewpoint of skin occlusion. Powder products where the film-forming ability cannot be effectively utilized has a problem that it is difficult to improve cosmetic retention.

The zinc oxide coated material described in Japanese Unexamined Patent Application Publication No. H9-227792 has an adsorption amount of oleic acid approximately the same as that of ordinary porous silica, and is not particularly excellent in adsorbing free fatty acids. An adsorption amount of artificial sebum is lower than that of porous silica beads, and a solidification time of fatty acid takes approximately 30 minutes, which poses a problem in that it is difficult to cope with oily skin or extremely oily skin.

In the interlayer metal inclusion compound described in Japanese Unexamined Patent Application Publication No. H10-87420, water-swellable clay minerals are used, the compound is produced by reaction in a sol state, and as understood from the description of the examples, the reaction is performed in a dilute solution. Therefore, such a method provides a high manufacturing cost per batch, which is economically very disadvantageous. Another disadvantage is that due to the reaction in the sol state, in the typical deliquification, washing, and drying processes, washing is extremely slow. This not only wastes considerable time but also causes strong aggregation in the resulting product not to achieve expected performance. Thus, there is a drawback that freeze-drying is an essential condition and a high cost is incurred. Another problem is that aluminum pillars are formed between the layers of the clay mineral, but due to batch-to-batch variations of the clay mineral and the state of pillar formation at this time, there is a large variation in the content of the intercalated oxide or hydroxide, and this makes it difficult to always obtain stable quality.

Japanese Unexamined Patent Application Publication No. 2002-20218 describes composite powders. It is reported that such composite powders having a hydroxyapatite layer on a surface of a base particle and a zinc oxide layer atop the hydroxyapatite layer have excellent sebum adsorption properties, antibacterial effects, and body odor component adsorption capability. Such composite powders are currently used as cosmetics with sebum adsorbing properties. However, there is an expectation for further improvement of cosmetic properties (cosmetic effects) such as improved cosmetic retention, suppression of "shine" phenomenon, and improved feel, and improved adsorption of unsaturated fatty acids and sebum.

The inventors of the present invention diligently conducted research in search of a suitable material for cosmetics that selectively adsorbs free fatty acids. As a result, the inventors found that by using particles including a biodegradable resin and a metal oxide, in which a ratio of an absorption amount of oleic acid to an absorption amount of triglyceride in the particles is 1.5 or more, particles that can selectively adsorb free fatty acids and are excellent in cosmetic retention are provided.

### [Oleic Acid Absorption Amount/Triglyceride Absorption Amount in Particles]

A ratio of an absorption amount of oleic acid to an absorption amount of triglyceride in the particles is 1.5 or more, preferably 1.5 or more and 7.0 or less, and more preferably 2.0 or more and 7.0 or less. When the value is 1.5 or more, free fatty acids are selectively adsorbed, and cosmetic retention can be improved. A larger value is more preferable as the free fatty acids can be selectively adsorbed.

Here, the oleic acid is a representative example of free fatty acids, and a neutral fat in which three molecules of the oleic acid are ester-bonded to glycerin is used as triglyceride. The aforementioned value serves as an index indicating the selective absorbency of the free fatty acids.

The absorption amount of the oleic acid in the particles is a value obtained by dividing the amount of the oleic acid [g] absorbed by the particles by the amount of particles [g]. The absorption amount of the triglyceride in the particles is a value obtained by dividing the amount of the triglyceride [g] absorbed by the particles by the amount of particles [g]. The ratio of the absorption amount of the oleic acid to the absorption amount of the triglyceride in the particles is a value obtained by dividing the "absorption amount of the oleic acid in particles" by the "absorption amount of the triglyceride in particles".

The maximum absorption amount of the oleic acid or the triglyceride in the particles can be measured according to the following procedure.

Specifically, a predetermined amount of the oleic acid or the triglyceride (hereinafter referred to as "each component") is added to a fixed amount of the particles and mixed for 15 minutes using a spatula. Thereafter, each component not absorbed by the particles is removed by pressing oil blotting paper (for example, high-quality oil blotting paper 90 mm x 90 mm, manufactured by Kai Corporation). Subsequently, the total mass of the particles and each component not removed by oil blotting paper but absorbed by the particles is measured. Subtracting the mass of the particles from the total mass then yields the mass of the oleic acid or the triglyceride absorbed by the particles. It is noted that to ensure that the absorption amount is saturated, the added amount of each component is set so that the mass of each component removed by oil blotting paper exceeds 5% by mass of the particles.

### [Relative Span Factor (R.S.F)]

"Relative Span Factor (R.S.F)" is defined as (D₉₀ - D₁₀)/D₅₀ and is an index representing the narrowness of particle size distribution. D₉₀ represents the cumulative 90% by number from the small particle side of the cumulative particle size distribution, D₅₀ represents the cumulative 50% by number from the small particle side of the cumulative particle size distribution, and D₁₀ represents the cumulative 10% by number from the small particle side of the cumulative particle size distribution. A smaller (R.S.F) value indicates a narrower particle size distribution.

Examples of methods for measuring R.S.F include, but are not limited to, a method using a concentrated particle analyzer based on a dynamic light scattering method ("FPAR-1000", manufactured by Otsuka Electronics Co., Ltd.) and a particle size distribution measuring device based on a laser diffraction/scattering method ("LA-960", manufactured by Horiba, Ltd.).

Examples of other indicators indicating the narrowness of the particle size distribution include, but are not limited to, the ratio of volume average particle size (Dv) to number average particle size (Dn). The ratio of volume average particle size (Dv) to number average particle size (Dn) is a value obtained by dividing the volume average particle size (Dv) by the number average particle size (Dn), and a smaller value indicates a narrower particle size distribution. The ratio of volume average particle size (Dv) to number average particle size (Dn) of the resin particles according to the present disclosure is preferably 1.00 or more and 1.50 or less, and more preferably 1.00 or more and 1.20 or less.

Examples of methods for measuring the volume average particle size (Dv) and the number average particle size (Dn) include, but are not limited to, a method using a laser diffraction/scattering particle size distribution analyzer (MICROTRAC MT3000II, manufactured by MicrotracBEL Corp.) or a particle size distribution analyzer based on a laser diffraction/scattering method ("LA-960", manufactured by Horiba, Ltd.).

### [Volume Average Particle Size]

The volume average particle size (Dv) of the particles is 3 µm or more and 50 µm or less. When the particles are larger than 50 µm, the contact area of free fatty acids with respect to the volume of the particles decreases, and thus, the absorption amount of free fatty acids decreases. On the other hand, when the volume average particle size (Dv) is 3 µm or more and 50 µm or less, the absorption amount of free fatty acids can be ensured, and the ratio of the absorption amount of the oleic acid to the absorption amount of the triglyceride may be 1.5 or more. From the viewpoint of tactile sensation and spreadability on the skin, the volume average particle size (Dv) is preferably 3 µm or more and 20 µm or less.

The mass ratio (B/M) of the biodegradable resin (B) to the metal oxide (M) in the particles is preferably 0.5/9.5 or more and 8/2 or less.

### <Biodegradable Resin>

The biodegradable resin is not particularly limited as long as such a resin has a biodegradability, but is preferably at least one resin selected from the group consisting of polyester resins, polyamide resins, and cellulose resins.

Examples of biodegradable polyester resins include, but are not limited to, polylactic acid; poly-ε-caprolactone; succinate polymers such as polyethylene succinate, polybutylene succinate, and polybutylene succinate adipate; polybutylene adipate terephthalate; polyhydroxyalkanoates such as polyhydroxypropionate, polyhydroxybutyrate, polyhydroxyvalerate, and poly(3-hydroxybutyrate-co-3-hydroxyhexanoate); and polyglycolic acid.

Examples of biodegradable polyamide resins include, but are not limited to, nylon 4.

Examples of biodegradable cellulose resins include, but are not limited to, hydroxypropyl cellulose, hydroxypropyl methylcellulose, and hypromellose acetate succinate.

These resins may be used alone or in combination of two or more types.

### - Polylactic Acid -

The weight average molecular weight Mw of polylactic acid is not particularly limited and can be appropriately selected according to the purpose, but the weight Mw is preferably 5,000 or more and 100,000 or less, more preferably 10,000 or more and 70,000 or less, and even more preferably 10,000 or more and 60,000 or less.

### - Polyglycolic Acid -

Polyglycolic acid is not particularly limited and can be appropriately selected according to the purpose. Examples thereof include, but are not limited to, a poly(lactic-co-glycolic acid), which is a copolymer having a constituent unit derived from lactic acid and a constituent unit derived from glycolic acid; a glycolic acid-caprolactone copolymer, which is a copolymer having a constituent unit derived from glycolic acid and a constituent unit derived from caprolactone; and a glycolic acid-trimethylene carbonate copolymer, which is a copolymer having a constituent unit derived from glycolic acid and a constituent unit derived from trimethylene carbonate. These may be used alone or in combination of two or more types. Among these, poly(lactic-co-glycolic acid) is preferable from the viewpoint of high biocompatibility, an ability to sustainedly elute the contained physiologically active substance, and a long-term storage of the contained physiologically active substance.

Examples of the poly(lactic-co-glycolic acid) include, but are not limited to, PURASORB PDLG5010, PURASORB PDLG75 10, PURASORB PDLG7507, PURASORB PDLG5002A, PURASORB PDLG5002, and PURASORB PDLG7502A (manufactured by Corbion); and RG502, RG502H, RG503, RG503H, RG504, and RG504H (manufactured by Sigma-Aldrich).

The weight average molecular weight of the poly(lactic-co-glycolic acid) is not particularly limited and can be appropriately selected according to the purpose, but the weight average molecular weight is preferably 2,000 or more and 250,000 or less, more preferably 2,000 or more and 100,000 or less, and particularly preferably 40,000 or more and 50,000 or less.

The molar ratio (L/G) of the constituent unit derived from lactic acid (L) to the constituent unit derived from glycolic acid (G) in the poly(lactic-co-glycolic acid) is not particularly limited and can be appropriately selected according to the purpose, but such a ratio is preferably 1/99 or more and 99/1 or less, more preferably 25/75 or more and 99/1 or less, even more preferably 30/70 or more and 90/10 or less, and particularly preferably 50/50 or more and 85/15 or less.

### - Polyhydroxyalkanoates -

Polyhydroxyalkanoates are not particularly limited and can be appropriately selected according to the purpose, but preferably is a poly(3-hydroxyalkanoate) polymer or copolymer consisting of a repeating unit represented by the following general formula (1).

[-CH(R)-CH₂CO-O-] General Formula (1)

In General Formula (1), R represents an alkyl group represented by -CₙH₂ₙ₊₁, and n is an integer from 1 to 15.

Examples of poly(3-hydroxyalkanoate) polymers or copolymers consisting of a repeating unit represented by General Formula (1) include, but are not limited to, polymers consisting of one type of repeating unit, or copolymers consisting of two or more types of repeating units, selected from the group consisting of 3-hydroxypropionate, 3-hydroxybutyrate, 3-hydroxyvalerate, 3-hydroxyhexanoate, 3-hydroxyheptanoate, 3-hydroxyoctanoate, 3-hydroxynonanoate, 3-hydroxydecanoate, 3-hydroxytetradecanoate, 3-hydroxyhexadecanoate, and 3-hydroxyoctadecanoate. Specifically, examples thereof include, but are not limited to, homopolymers of the 3-hydroxyalkanoate, or copolymers consisting of two or more types of 3-hydroxyalkanoates with different n values, or mixtures obtained by blending two or more selected from the group consisting of the homopolymers and the copolymers. Among these, homopolymers, copolymers, and mixtures thereof, including repeating units selected from the group consisting of 3-hydroxybutyrate repeating units where n = 1, 3-hydroxyvalerate repeating units where n = 2, 3-hydroxyhexanoate repeating units where n = 3, 3-hydroxyoctanoate repeating units where n = 5, and 3-hydroxyoctadecanoate repeating units where n = 15, are preferred. More preferred are copolymers including 3-hydroxybutyrate repeating units and at least one repeating unit selected from the group consisting of 3-hydroxyvalerate, 3-hydroxyhexanoate, and 3-hydroxyoctanoate.

When using a copolymer of 3-hydroxybutyrate and 3-hydroxyhexanoate, the ratio of 3-hydroxybutyrate to 3-hydroxyhexanoate is not particularly limited and can be appropriately selected according to the purpose; however, from the viewpoint of suppressing an increase in viscosity of the particle composition liquid due to a higher ratio of 3-hydroxybutyrate and achieving good droplet discharge in a droplet discharge step, the molar ratio (HB/HH) of 3-hydroxybutyrate (HB) to 3-hydroxyhexanoate (HH) is preferably 80/20 or more and 94/6 or less, and more preferably 80/20 or more and 90/10 or less.

The weight average molecular weight Mw of the polyhydroxyalkanoate is not particularly limited and can be appropriately selected according to the purpose. However, to suppress an increase in viscosity of the particle composition liquid due to a larger molecular weight and to achieve good droplet discharge in a droplet discharge step, the weight Mw is preferably 2,000 or more and 1,000,000 or less, and more preferably 2,000 or more and 600,000 or less.

### <Metal Oxide Particles>

The metal oxide is not particularly limited and can be appropriately selected according to the purpose. Examples thereof include, but are not limited to, single-component powders such as silica, alumina, zinc oxide, titanium oxide, and cerium oxide; bismuth oxychloride, and barium sulfate. These may be used alone, in combination of two or more, or in the form of a metal oxide composite.

Examples of the metal oxide composite include, but are not limited to, multilayer composites such as silica-titanium oxide, silica-zinc oxide, silica-titanium oxide-silica, silica-cerium oxide-silica, and silica-zinc oxide-silica; pearl pigments such as titanium mica, colored titanium mica, titanium oxide-barium sulfate, titanium oxide-talc, zinc oxide-mica, zinc oxide-talc, and bismuth oxychloride-mica; pearl pigments surface-treated with aluminum hydroxide, aluminum oxide, magnesium hydroxide, magnesium oxide, silica treatment, or barium sulfate treatment; and hard capsules such as titanium oxide-encapsulated acrylic/methacrylic resin (PMMA), zinc oxide-encapsulated PMMA, and cerium oxide-encapsulated PMMA.

Among these, zinc oxide is preferable due to its good reactivity with free fatty acids.

The shape of the metal oxide is preferably particulate because such a shape provides a large contact area with free fatty acids. The volume average particle size (Dv) of the metal oxide is preferably 10 nm or more and 200 nm or less, and more preferably 10 nm or more and 100 nm or less.

### <Other Components>

The particles may optionally be combined with other components such as physiologically active substances and dispersion stabilizers, and can be used as functional particles depending on various applications. As will be described later, the particles can be used as particles for cosmetics by combining such particles with cosmetic components.

Functional particles are not particularly limited and can be appropriately selected according to the purpose. Examples thereof include, but are not limited to, immediate-release particles, sustained-release particles, pH-dependent release particles, pH-independent release particles, enteric-coated particles, controlled-release coated particles, and nanocrystal-containing particles.

### <<Physiologically Active Substances>>

The physiologically active substances are not particularly limited and can be appropriately selected according to the purpose. Examples thereof include, but are not limited to, alcohols, fatty alcohols, and polyols, aldehydes, alkanolamines, alkoxylated alcohols (for example, polyethylene glycol derivatives of alcohols and fatty alcohols), alkoxylated amides, alkoxylated amines, alkoxylated carboxylic acids, amides containing salts (for example, ceramides), amines, amino acids including salts and alkyl-substituted derivatives, esters, alkyl-substituted and acyl derivatives, polyacrylic acids, acrylamide copolymers, adipic acid copolymers, aminosilicones, biological polymers and their derivatives, butylene copolymers, carbohydrates (for example, polysaccharides, chitosan, and derivatives thereof), carboxylic acids, carbomers, esters, ethers, and polymer ethers (for example, PEG derivatives and PPG derivatives), glyceryl esters and derivatives thereof, halogen compounds, heterocyclic compounds containing salts, hydrophilic colloids and derivatives containing salts and gums (for example, cellulose derivatives, gelatin, xanthan gum, and natural rubbers), imidazolines, inorganic substances (clay, TiO₂, and ZnO), ketones (for example, camphor), isethionates, lanolin and derivatives thereof, organic salts, phenols containing salts (for example, parabens), phosphorus compounds (for example, phosphate derivatives), polyacrylates and acrylate copolymers, proteins and enzyme derivatives (for example, collagen), synthetic polymers containing salts, siloxanes and silanes, sorbitan derivatives, sterols, sulfonic acids and derivatives thereof, and waxes. These may be used alone or in combination of two or more types.

### <<Dispersion Stabilizer>>

The dispersion stabilizer is not particularly limited and can be appropriately selected according to the purpose. Examples thereof include, but are not limited to, alkaline earth metal salts; and sparingly water-soluble inorganic compounds such as calcium carbonate and calcium phosphate. Among these, calcium carbonate surface-treated with a silane coupling agent is preferable due to its high compatibility with biodegradable resins and excellent dispersion stability. The content of the alkaline earth metal component in the particles is preferably less than 10 mass ppm with respect to the total mass of the particles, from the viewpoint of suppressing the aggregation of particles due to adsorption of atmospheric moisture onto the dispersion stabilizer.

Examples of methods for measuring an amount of alkaline earth metal components contained in particles include the following method. 1.0 g of the particles to be measured is precisely weighed into a crucible and incinerated by heating at 450°C for three hours using an electric furnace. The incinerated particles are dissolved in 2 mL of concentrated hydrochloric acid, brought to a constant volume of 50 mL with ultrapure water to prepare a measurement sample, and thereafter, the amount of alkaline earth metal components can be measured using a multi-type ICP emission spectrometer ("ICPE-9000", manufactured by Shimadzu Corporation).

### (Cosmetics)

The cosmetics of the present embodiment contain the particles according to the present embodiment and optionally contain other components.

### <Other Components>

As other components, well-known cosmetic components can be appropriately selected. Examples thereof include, but are not limited to, physiologically active substances, dispersion stabilizers, pigments, oily bases, emulsifiers, preservatives, and fragrances.

For example, a foundation can be produced by mixing the particles according to the present embodiment with pigments, oily bases, emulsifiers, preservatives, fragrances, and the like.

Pigments are not particularly limited and well-known cosmetic components can be appropriately selected according to the purpose. Examples thereof include, but are not limited to, inorganic pigments, organic pigments, pearl pigments, and metal powder pigments.

Oily bases are not particularly limited and well-known cosmetic components can be appropriately selected according to the purpose. Examples thereof include, but are not limited to, ester oils, hydrocarbon oils, silicone oils, higher fatty acids, alkyl glyceryl ethers, liquid oils, solid oils, semi-solid oils, and oil-soluble agent.

Emulsifiers are not particularly limited and well-known cosmetic components can be appropriately selected according to the purpose. Examples thereof include, but are not limited to, anionic surfactants, cationic surfactants, nonionic surfactants, and amphoteric surfactants.

### [Applications]

The particles according to the present embodiment are useful as raw materials for cosmetics. Specific cosmetics include, but are not limited to, foundations, blushers, eye shadows, cleansers, face wash creams, sunscreen creams, antiperspirants, pre-shave lotions, after-shave lotions, face powders, toners, packs, massage creams, emulsions, moisturizing creams, serums, lipsticks, eyeliners, nail enamels, soaps, bath additives, shampoos, conditioners, hair treatments, sun oils, bleaching creams, depilatory creams, insect repellent lotions, insect repellent sprays, hair liquids, pomades, hair coloring agents, eau de cologne, shampoos, conditioners, and hair styling products. Among these, foundations, blushers, eye shadows, cleansers, face wash creams, sunscreen creams, antiperspirants, and pre-shave lotions are preferable because free fatty acids derived from sebum secreted from the skin are selectively adsorbed and they significantly contribute to excellent cosmetic retention.

### (Method and Apparatus for Producing Particles)

A method for producing particles according to the present embodiment includes a step of discharging a composition containing a biodegradable resin, a metal oxide, and a solvent into a gas in a form of droplets (droplet discharge step), and a step of removing the solvent from the droplets to granulate particles (granulation step), and may further include other steps optionally.

A particle manufacturing apparatus according to the present embodiment includes a droplet discharger that discharges a composition containing a biodegradable resin, a metal oxide, and a solvent into a gas in a form of droplets, and a granulator that removes the solvent from the droplets to granulate particles, and also includes other means optionally.

According to the method for producing particles and the particle manufacturing apparatus according to the present embodiment, the particles according to the present embodiment can be suitably produced, and particles that can selectively adsorb free fatty acids and provide excellent cosmetic retention can be suitably manufactured.

### <Droplet Discharge Step and Droplet Discharger>

The droplet discharge step is a step of discharging a composition containing a biodegradable resin, a metal oxide, and a solvent into a gas in a form of droplets, and can be suitably carried out by the droplet discharger.

The droplet discharger discharges a composition containing a biodegradable resin, a metal oxide, and a solvent into a gas in a form of droplets, and examples thereof include a nozzle including a composition storage container. The droplet discharger preferably employs a method of forming droplets by bringing the composition into contact with a gas to form particles.

The composition storage container is a container that contains a composition containing a metal oxide and a solvent, and may include a stirrer such as a stirring fin or a magnetic stirrer.

The composition storage container may be flexible or inflexible. The material of the composition storage container is not particularly limited and can be appropriately selected depending on the purpose. For example, the container may be formed of resin or metal. The structure of the composition storage container is not particularly limited and can be appropriately selected depending on the purpose. For example, the structure may be a sealed structure or a non-sealed structure.

A connector that connects the nozzle and the composition storage container is not particularly limited as long as the connector allows the composition to be supplied from the composition storage container to the nozzle, and can be selected appropriately depending on the purpose, and examples thereof include tubing (pipes, tubes, or the like).

### -Composition-

The composition contains a biodegradable resin, a metal oxide, and a solvent, and further contains other components optionally.

The biodegradable resin and the metal oxide in the composition, and the mass ratio (B/M) of the biodegradable resin (B) to the metal oxide (M) can be appropriately selected from the items described for the particles according to the present embodiment. The mass ratio (B/M) is preferably 0.5/9.5 or more and 8/2 or less.

In one embodiment, the composition and the solvent are preferably substantially free of surfactants. In particular, when the produced particles are used in applications such as pharmaceutical compositions, functional foods, and functional cosmetics, it is preferable that the particles are substantially free of surfactants to improve safety. Here, the case where the composition does not substantially contain surfactants includes, for example, a case where the content of the surfactant in the composition and the solvent is equal to or less than the detection limit that cannot be detected by liquid chromatography, and a case where the composition and the solvent do not contain a surfactant.

### --Solvent-

Examples of the solvents include water, aliphatic halogenated hydrocarbons (for example, dichloromethane, dichloroethane, or chloroform), alcohols (for example, methanol, ethanol, propanol, 3-methoxy-3-methyl-1-butanol, or 2,2,2-trifluoroethanol), ketones (for example, acetone, or methyl ethyl ketone), ethers (for example, diethyl ether, dibutyl ether, or 1,4-dioxane), aliphatic hydrocarbons (for example, n-hexane, cyclohexane, or n-heptane), aromatic hydrocarbons (for example, benzene, toluene, or xylene), organic acids (for example, acetic acid, or propionic acid), esters (for example, ethyl acetate, or 3-methoxy-3-methyl-1-butyl acetate), amides (for example, dimethylformamide or dimethylacetamide), and mixed solvents thereof. These may be used alone or in combination of two or more types. Among these, ketones, ethers, alcohols, or mixed solvents thereof are preferred from the viewpoint of solubility, and acetone, 1,3-dioxolane, ethanol, 2-propanol, or mixed solvents thereof are more preferred.

In one embodiment, by using a mixed solvent in which a poor solvent is added to a good solvent for the biodegradable resin, it is possible to change the particle shape of the resulting particles from a spherical shape to a porous shape. From the viewpoint of increasing the contact area between the particles and the free fatty acids, the solvent is preferably a mixture of a good solvent and a poor solvent for the biodegradable resin. The mass ratio of the good solvent to the poor solvent (good solvent/poor solvent) is preferably 40/60 or more and 90/10 or less, and more preferably 50/50 or more and 90/10 or less.

The content of the solvent is preferably 70% by mass or more and 99.5% by mass or less and more preferably 80% by mass or more and 99% by mass or less of the total mass of the composition. When the content is 70% by mass or more and 99.5% by mass or less, production stability is improved in terms of the solubility of the particulate material and the liquid viscosity.

In one embodiment, the biodegradable resin can be used as an emulsion without using a good solvent for the biodegradable resin. A desired composition can be obtained by mixing a dispersion of nanoparticles of a biodegradable resin in water or a poor solvent for the biodegradable resin with a metal oxide. It is noted that the particle size of the nanoparticles of the biodegradable resin is preferably 10 nm or more and 800 nm or less.

The viscosity of the composition is not particularly limited and can be appropriately selected depending on the purpose, but is preferably 0.5 mPa·s or more and 15.0 mPa·s or less, and more preferably 0.5 mPa·s or more and 10.0 mPa·s or less. When the viscosity of the composition is 0.5 mPa·s or more and 15.0 mPa·s or less, suitable discharge can be achieved by the above-mentioned droplet discharger.

It is noted that the viscosity can be measured, for example, using a viscoelasticity measuring device (device name: MCR rheometer, manufactured by AntonPaar) under conditions of 25°C and a shear rate of 10 s⁻¹.

The surface tension of the composition is not particularly limited and can be appropriately selected depending on the purpose, but is preferably 10 mN/m or more and 60 mN/m or less, and more preferably 20 mN/m or more and 50 mN/m or less. When the surface tension of the composition is 0.5 mPa·s or more and 15.0 mPa·s or less, the droplets can be suitably discharged by the above-mentioned droplet discharger.

The surface tension can be measured, for example, using a handy surface tensiometer (device name: PocketDyne, manufactured by KRUSS) by the maximum bubble pressure method under conditions of 25°C and a lifetime of 1,000 ms.

### <Granulation Step and Granulator>

The granulation step is a step of removing the solvent from the droplets to granulate particles, and can be suitably carried out by a granulator.

The granulator granulates particles by removing the solvent from the droplets, and examples thereof include a space former that forms a space for evaporating the solvent from the droplets.

The granulation step is preferably carried out in a gas, specifically while the droplets discharged into the gas in the droplet discharge step are flying in the gas.

Methods for removing the solvent from the droplets include, for example, adding a dry air flow to evaporate the solvent, creating a reduced pressure atmosphere using a pressure reducing device, heating, and applying a chemical reaction to remove the solvent, and combinations thereof.

The dry air flow may contain chemicals that promote drying of the droplets. The state of the dry air flow is not limited, and may be laminar flow, swirling flow, or turbulent flow. The type of gas constituting the dry air flow is not particularly limited and can be appropriately selected depending on the purpose. Air or a non-flammable gas such as nitrogen may be used. It is also preferable to appropriately adjust a temperature, a vapor pressure, a type of gas, or the like of the gas used for drying.

As long as the collected particles remain in a solid state, the solvent does not have to be completely evaporated, and a drying step may be added as a separate step after collection. Furthermore, if the amount of residual solvent contained in the obtained particles is large, it is preferable to carry out secondary drying optionally to reduce such an amount. For the secondary drying, a commonly known drying method such as fluidized bed drying or vacuum drying can be used.

The liquid to be sprayed may be heated to a temperature equal to or higher than the melting point of the biodegradable resin, and after spraying, the biodegradable resin in the droplets may be solidified by the cooling effect caused by evaporation of the solvent to form particles.

The content of the solvent in the particles is preferably 5% by mass or less, as determined by gas chromatography, as the amount of residual solvent in the particles. When the content is 5% by mass or less, the adverse effect of the residual solvent, that is, the effect that particles tend to stick together to cause aggregation and worsen the particle size distribution of the particles, can be reduced, and the R.S.F. is a good value of 1.2 or less.

The amount of the residual solvent in the particles can be measured by adding 2 parts by mass of 2-propanol to 1 part by mass of particles to be measured, dispersing the particles ultrasonically for 30 minutes, then storing the mixture in a refrigerator (5°C) for one day or more to extract the solvent in the particles, and analyzing the supernatant by gas chromatography (GC-14A, manufactured by Shimadzu Corporation) to quantify the amount of solvent in the particles.

### <Other Steps and Other Means>

The other steps are not particularly limited and can be appropriately selected depending on the purpose. Examples thereof include a particle collection step and a classification step.

The other means are not particularly limited and can be appropriately selected depending on the purpose. Examples thereof include a particle collector and a classifier.

The particle collection step is a step of collecting the particles obtained in the granulation step, and can be suitably carried out by the particle collector. The particle collector is not particularly limited and can be appropriately selected depending on the purpose. Examples thereof include cyclone collection and back filters.

In producing particles containing at least two types of substrates, one of which is biased toward the surface side, this type of particles can be formed in the granulation step by appropriately selecting the type of the substrate contained in the composition.

In the granulation step, to form particles in which one of the at least two types of substrates is contained biased toward the surface side, the contact angles of the at least two types of substrates are made different from each other. This increases the interaction between the substrates when the solvent is evaporated from the droplets in the granulation step.

At this time, the contact angles of at least two types of substrates are different from each other, and thus, the substrates are likely to undergo phase separation from each other. As a result, one of the at least two types of substrates is unevenly contained on the surface side, and as the solvent subsequently evaporates, particles solidified in this state are formed. This method makes it possible to form particles in which one of at least two types of substrates is contained unevenly on the surface side, in only one step.

The classification step is a step in which the particles obtained in the granulation step are classified to obtain particles having a uniform size, and can be suitably carried out by classifier. The classifier is not particularly limited and can be appropriately selected depending on the purpose, and examples thereof include filters and sieves.

### [Examples]

Examples of the present disclosure will be described below, but the present disclosure is not limited to these examples in any way.

### (Example 1-1)

### - Preparation of Formulation A -

A powder of polylactic acid (PLA-R-001FL, manufactured by Nagase & Co., Ltd.) and zinc oxide (FINEX-50, Sakai Chemical Industry Co., Ltd.) mixed in a mass ratio of 2:8 was mixed with a solvent of acetone and 2-propanol in a mass ratio of 1:1 to prepare formulation A with a solids concentration of 15% by mass.

### - Granulation of Particles 1 -

Using the formulation A, particles 1 were granulated by spray drying means (Tri-Spire nozzle, manufactured by GF Co., Ltd.) under the following particle producing conditions.

The volume average particle size (Dv) of the obtained particles 1 was 5.4 µm, and (R.S.F.) was 0.80. The value of the absorption amount of the oleic acid/the absorption amount of the triglyceride in the particles was 5.2.

### [Particle Producing Conditions]

- Spray drying means: Tri-Spire nozzle (GF Co., Ltd.)
- Granulation equipment: Spray dryer GS310 (Yamato Scientific Co., Ltd.)
- Formulation feed rate (Tri-Spire nozzle spray conditions): 15 g/min
- Compressed air volume (Tri-Spire nozzle injection conditions): 0.6 MPa, 30 NL/min
- Dry air flow rate: 50 m³/h
- Dry air flow temperature: 40°C

### [Measurement of Volume Average Particle Size (Dv) and (R.S.F.)]

The volume average particle size (Dv), D₁₀, D₅₀, and D₉₀ of the obtained particles were measured using a particle size distribution measuring device (LA-960, manufactured by Horiba, Ltd.) based on the laser diffraction/scattering method. Next, (R.S.F.) was calculated according to the calculating formula (R.S.F.) = (D₉₀ - D₁₀)/D₅₀. The measurement sample was a dispersion obtained by adding 0.1 g of DRIWEL K (dry well, manufactured by Fujifilm Corporation) and 5.0 g of water to 0.003 g of particles to be measured and ultrasonically dispersing the mixture for three minutes.

### - Measurement Conditions -

- Transmittance (R): 85 to 95%
- Transmittance (B): 70 to 90%
- Algorithm options: Standard mode

### [Measurement of Oleic Acid Absorption Amount/Triglyceride Absorption Amount in Particles]

The maximum absorption amount of oleic acid or triglyceride in the particles was measured according to the following procedure.

Specifically, a predetermined amount of oleic acid or triglyceride (hereinafter referred to as "each component") was added to 0.4 g of particles, and the mixture was mixed with a spatula for 15 minutes. Thereafter, each component not absorbed by the particles was removed by pressing oil blotting paper (high-quality oil blotting paper 90 mm x 90 mm, manufactured by Kai Corporation). The total mass of the particles and each component that could not be removed with the oil blotting paper and was absorbed into the particles was then measured, and the mass of the particles was subtracted to calculate the mass of oleic acid or triglyceride absorbed into the particles. To ensure that the absorption was saturated, the amount of each component added was set so that the mass of each component removed by the oil blotting paper exceeded 5% by mass of the particles.

### (Example 1-2)

Particles 2 were granulated in the same manner as in Example 1-1, except that formulation B was prepared by changing the mass ratio of the biodegradable resin to the metal oxide in the formulation A from 2:8 to 5:5 in Example 1-1.

### (Example 1-3)

The particles 3 were granulated in the same manner as in Example 1-1, except that formulation C was prepared by changing the mass ratio of the biodegradable resin to the metal oxide in the formulation A from 2:8 to 8:2 in Example 1-1.

### (Example 1-4)

### - Granulation of Particles 4 -

Using the formulation B, particles 4 were granulated by spray drying means (rotary disk atomizer, manufactured by Ohkawara Kakohki Co., Ltd.) under the following particle producing conditions. The volume average particle size (Dv) of the obtained particles 4 was 18.9 µm, and (R.S.F.) was 0.75. The value of the absorption amount of the oleic acid/the absorption amount of the triglyceride in the particles was 3.5.

### [Particle Producing Conditions]

- Spray drying means: Rotary disk atomizer (Ohkawara Kakohki Co., Ltd.)
- Granulation equipment: Spray dryer L-8 (Ohkawara Kakohki Co., Ltd.)
- Rotary disc atomizer rotation speed: 10,000 rpm
- Formulation feed rate: 2 kg/hour
- Carrying air flow rate: 50 m³/h
- Dry air flow temperature: 50°C

### (Example 1-5)

Particles 5 were granulated in the same manner as in Example 1-2, except that the biodegradable resin in the formulation A was changed from polylactic acid to polybutylene adipate terephthalate (PBAT, manufactured by Miyako Chemical Co., Ltd.) to prepare formulation D in Example 1-2.

### (Example 1-6)

Particles 6 were granulated in the same manner as in Example 1-2, except that the biodegradable resin in the formulation A was changed from polylactic acid to polyhydroxyalkanoate (BP350-15, manufactured by BLUPHA Co., Ltd) to prepare formulation E in Example 1-2.

### (Example 1-7)

Particles 10 were granulated in the same manner as in Example 1-1, except that formulation H was prepared by changing the mass ratio of the biodegradable resin to the metal oxide in the formulation A from 2:8 to 0.5:9.5 in Example 1-1.

### (Example 1-8)

Particles 11 were granulated in the same manner as in Example 1-1, except that formulation I was prepared by changing the biodegradable resin in the formulation A from polylactic acid to hypromellose acetate succinate (HPMCAS-HG, Shin-Etsu Chemical Co., Ltd.) in Example 1-1.

### (Comparative Example 1-1)

### - Preparation of Formulation F -

Zinc oxide (FINEX-50, Sakai Chemical Industry Co., Ltd.) was mixed with a solvent of acetone and 2-propanol in a mass ratio of 1:1 to prepare formulation F with a solids concentration of 15% by mass.

### - Granulation of Particles 7 -

Using the obtained formulation F, particles 7 were granulated in the same manner as in Example 1. The volume average particle size (Dv) of the obtained particles 7 was 1.2 µm, and (R.S.F.) was 1.15. The value of the absorption amount of the oleic acid/the absorption amount of the triglyceride in the particles was 1.0.

### (Comparative Example 1-2)

### - Preparation of Formulation G -

Polylactic acid (PLA-R-001FL, manufactured by Nagase & Co., Ltd.) was mixed with a solvent of acetone and 2-propanol in a mass ratio of 1:1 to prepare formulation G with a solids concentration of 15% by mass.

### - Granulation of Particles 8 -

Using the obtained formulation G, particles 8 were granulated in the same manner as in Example 1. The volume average particle size (Dv) of the obtained particles 8 was 5.4 µm, and (R.S.F.) was 0.91. The value of the absorption amount of the oleic acid/the absorption amount of the triglyceride in the particles was 1.0.

### (Comparative Example 1-3)

### - Granulation of Particles 9 -

Using the formulation B, particles 9 were granulated by spray drying means (one-fluid nozzle, manufactured by Ohkawara Kakohki Co., Ltd.) under the following particle producing conditions. The volume average particle size (Dv) of the obtained particles 9 was 82.2 µm, and (R.S.F.) was 2.5. The value of the absorption amount of the oleic acid/the absorption amount of the triglyceride in the particles was 1.1.

### [Particle Producing Conditions]

- Spray drying means: One-fluid nozzle (Ohkawara Kakohki Co., Ltd.)
- Granulation equipment: Spray dryer L-8 (Ohkawara Kakohki Co., Ltd.)
- Formulation feed rate: 2 kg/hour
- Carrying air flow rate: 50 m³/h
- Dry air flow temperature: 50°C

Table 1 presents the producing conditions for the particles 1 to 11. It is noted that in Table 1, "PLA" stands for "polylactic acid". "PBAT" stands for "polybutylene adipate terephthalate". "PHBH" stands for "poly(3-hydroxybutyrate-co-3-hydroxyhexanoate)".

**Table 1**

| | Type | Biodegradable resin (B) | Metal oxide (M) | B:M [Mass ratio] | Granulation method |
|---|---|---|---|---|---|
| Example 1-1 | Particles 1 | PLA | Zinc oxide | 2:8 | Tri-Spire nozzle |
| Example 1-2 | Particles 2 | PLA | Zinc oxide | 5:5 | Tri-Spire nozzle |
| Example 1-3 | Particles 3 | PLA | Zinc oxide | 8:2 | Tri-Spire nozzle |
| Example 1-4 | Particles 4 | PLA | Zinc oxide | 5:5 | Rotary atomizer |
| Example 1-5 | Particles 5 | PBAT | Zinc oxide | 5:5 | Tri-Spire nozzle |
| Example 1-6 | Particles 6 | PHBH | Zinc oxide | 5:5 | Tri-Spire nozzle |
| Example 1-7 | Particles 10 | PLA | Zinc oxide | 0.5:9.5 | Tri-Spire nozzle |
| Example 1-8 | Particles 11 | HPMCAS | Zinc oxide | 2:8 | Tri-Spire nozzle |
| Comparative Example 1-1 | Particles 7 | - | Zinc oxide | 0:10 | Tri-Spire nozzle |
| Comparative Example 1-2 | Particles 8 | PLA | - | 10:0 | Tri-Spire nozzle |
| Comparative Example 1-3 | Particles 9 | PLA | Zinc oxide | 5:5 | One-fluid nozzle |

### (Example 2-1)

### <Preparation of Powder Foundation>

A mixture was prepared by mixing 10 parts by mass of the particles 1 manufactured in Example 1, 21 parts by mass of sericite, 56 parts by mass of muscovite, 0.6 parts by mass of red iron oxide, 1 part by mass of yellow iron oxide, and 0.1 parts by mass of black iron oxide serving as pigments using a Henschel mixer. Separately, 10 parts by mass of cetyl 2-ethylhexanoate serving as an oily base, 1 part by mass of sorbitan sesquioleate serving as an emulsifier, and 0.2 parts by mass of a preservative were mixed and dissolved to prepare a lysate. The prepared mixture and lysate were mixed uniformly, 0.1 parts by mass of fragrance was further added and mixed uniformly, and then the mixture was pulverized and passed through a sieve to prepare a foundation material. This foundation material was compressed and molded into a metal dish to prepare the powder foundation of Example 1.

### (Examples 2-2 to 2-8 and Comparative Examples 2-1 to 2-3)

Powder foundations of Examples 2-2 to 2-8 and Comparative Examples 2-1 to 2-3 were prepared in the same manner as in Example 2-1, except that the particles 1 was changed to the particles 2 to 6, 10 to 11, and 7 to 9, respectively in Example 2-1.

### (Evaluation)

Ten panelists used each of the obtained powder foundations for three days and evaluated how well the makeup lasted (how long the cosmetics was retained). The evaluation criteria were based on the following five levels, and the average of the evaluation results from the ten panelists was calculated to obtain the evaluation result. An average value of 4.0 or more was considered to be acceptable. The results are presented in Table 2.

### - Evaluation Criteria -

5: The cosmetic retention is excellent.
4: The cosmetic retention is good.
3: The cosmetic retention is fair.
2: The cosmetic retention is poor.
1: The cosmetic retention is very poor.

**Table 2**

| | Particles | | | Evaluation result | |
|---|---|---|---|---|---|
| | Type | Volume average particle size Dv [µm] | R.S.F. | Absorption amount of oleic acid/absorption amount of triglyceride | Cosmetic retention |
| Example 2-1 | Particles 1 | 5.4 | 0.80 | 5.2 | 4.3 |
| Example 2-2 | Particles 2 | 6.4 | 0.83 | 4.0 | 4.3 |
| Example 2-3 | Particles 3 | 6.5 | 0.90 | 2.0 | 4.1 |
| Example 2-4 | Particles 4 | 18.9 | 0.75 | 3.5 | 4.0 |
| Example 2-5 | Particles 5 | 6.2 | 0.89 | 4.2 | 4.2 |
| Example 2-6 | Particles 6 | 6.6 | 0.91 | 4.1 | 4.2 |
| Example 2-7 | Particles 10 | 6.7 | 0.85 | 4.5 | 4.3 |
| Example 2-8 | Particles 11 | 6.1 | 0.82 | 5.1 | 4.3 |
| Comparative Example 2-1 | Particles 7 | 1.2 | 1.15 | 1.0 | 3.4 |
| Comparative Example 2-2 | Particles 8 | 5.4 | 0.91 | 1.0 | 3.2 |
| Comparative Example 2-3 | Particles 9 | 82.2 | 2.5 | 1.1 | 2.9 |

The results of Examples 2-1 to 2-8 present that the use of cosmetics containing particles that contain a biodegradable resin and a metal oxide, in which the value of the absorption amount of the oleic acid/the absorption amount of the triglyceride in the particles is 1.5 or more, the volume average particle size is 3 µm or more and 50 µm or less, and the Relative Span Factor (R.S.F.) is 1.2 or less, results in excellent cosmetic retention.

On the other hand, the results of Comparative Examples 2-1 to 2-3 present that if the value of the absorption amount of the oleic acid/the absorption amount of the triglyceride in the particles is 1.5 or less, the cosmetic retention is poor.

The present disclosure has been described above based on embodiments. However, the present disclosure is not limited to the requirements indicated in the above-described embodiments. These aspects can be changed without departing from the gist of the present disclosure, and can be appropriately determined depending on the application thereof.

It is noted that aspects of the embodiments of the present disclosure are as follows, for example.

### Aspect 1

Particles containing a biodegradable resin and a metal oxide, in which a ratio of an absorption amount of oleic acid to an absorption amount of triglyceride in the particles is 1.5 or more.

### Aspect 2

The particles according to Aspect 1, in which the particles have a volume average particle size of 3 µm or more and 50 µm or less and a Relative Span Factor (R.S.F.) of 1.2 or less.

### Aspect 3

The particles according to Aspect 1 or 2, in which a mass ratio (B/M) of the biodegradable resin (B) to the metal oxide (M) is 0.5/9.5 or more and 8/2 or less.

### Aspect 4

The particles according to any one of Aspects 1 to 3, in which the metal oxide comprises zinc oxide.

### Aspect 5

The particles according to any one of Aspects 1 to 4, in which the metal oxide has a volume average particle size of 10 nm or more and 200 nm or less.

### Aspect 6

The particles according to any one of Aspects 1 to 5, in which the biodegradable resin includes at least one of a polylactic acid and a poly(lactic-co-glycolic acid).

### Aspect 7

The particles according to any one of Aspects 1 to 6, in which the biodegradable resin includes polyhydroxyalkanoate.

### Aspect 8

The particles according to Aspect 7, in which the polyhydroxyalkanoate is poly(3-hydroxybutyrate-co-3-hydroxyhexanoate).

### Aspect 9

The particles according to any one of Aspects 1 to 8, in which the biodegradable resin includes polybutylene adipate terephthalate.

### Aspect 10

The particles according to any one of Aspects 1 to 9, in which the biodegradable resin includes hypromellose acetate succinate.

### Aspect 11

Cosmetics containing the particles according to any one of Aspects 1 to 10.

### Aspect 12

The cosmetics according to Aspect 11, in which the cosmetics include a foundation, a blusher, an eye shadow, a cleanser, a face wash cream, a sunscreen cream, an antiperspirant, or a pre-shave lotion.

### Aspect 13

A method for producing the particles according to any one of Aspects 1 to 10, including discharging a mixed liquid containing the biodegradable resin, the metal oxide, and a solvent into a gas in a form of droplets, and removing the solvent from the droplets to granulate particles.

### Aspect 14

The method for producing the particles according to Aspect 13, in which the solvent is a mixture of a good solvent for the biodegradable resin and a poor solvent for the biodegradable resin.

The particles according to any one of Aspects 1 to 10, the cosmetics according to Aspect 11 or 12, and the method for producing resin particles according to Aspect 13 or 14 can solve various problems in the related art and achieve the object of the present disclosure.

Any one of the above-described operations may be performed in various other ways, for example, in an order different from the one described above.

## Claims

1. Particles comprising:
a biodegradable resin; and
a metal oxide,
wherein a ratio of an absorption amount of oleic acid to an absorption amount of triglyceride in the particles is 1.5 or more.

2. The particles according to claim 1, wherein the particles have a volume average particle size of 3 µm or more and 50 µm or less and a Relative Span Factor of 1.2 or less.

3. The particles according to claim 1 or 2, wherein a mass ratio of the biodegradable resin to the metal oxide is 0.5/9.5 or more and 8/2 or less.

4. The particles according to any one of claims 1 to 3, wherein the metal oxide comprises zinc oxide.

5. The particles according to any one of claims 1 to 4, wherein the metal oxide has a volume average particle size of 10 nm or more and 200 nm or less.

6. The particles according to any one of claims 1 to 5, wherein the biodegradable resin includes at least one of a polylactic acid and a poly(lactic-co-glycolic acid).

7. The particles according to any one of claims 1 to 6, wherein the biodegradable resin includes polyhydroxyalkanoate.

8. The particles according to claim 7, wherein the polyhydroxyalkanoate is poly(3-hydroxybutyrate-co-3-hydroxyhexanoate).

9. The particles according to any one of claims 1 to 8, wherein the biodegradable resin includes polybutylene adipate terephthalate.

10. The particles according to any one of claims 1 to 9, wherein the biodegradable resin includes hypromellose acetate succinate.

11. Cosmetics comprising the particles according to any one of claims 1 to 10.

12. The cosmetics according to claim 11, wherein the cosmetics include a foundation, a blusher, an eye shadow, a cleanser, a face wash cream, a sunscreen cream, an antiperspirant, or a pre-shave lotion.

13. A method for producing the particles according to any one of claims 1 to 10, the method comprising:
discharging a mixed liquid containing the biodegradable resin, the metal oxide, and a solvent into a gas in a form of droplets; and
removing the solvent from the droplets to granulate particles.

14. The method according to claim 13, wherein the solvent is a mixture of a good solvent for the biodegradable resin and a poor solvent for the biodegradable resin.
